# EUROPEAN PATENT APPLICATION

(11) **EP 2 008 593 A1**
(43) Date of publication of application: **31.12.2008**
(21) Application number: 08011485.3
(22) Date of filing: 24.06.2008
(51) Int. Cl.: A61B 17/02

(54) **Skin traction surgical separator**

(30) Priority: 25.06.2007 ES 200701372 U
(71) Applicant: Piñero Madrona, Antonio, 30007 Murcia (ES)
(72) Inventor: Piñero Madrona, Antonio, 30007 Murcia (ES)
(74) Representative: Soler Lerma, Santiago

(57) **Abstract**

1. A skin traction surgical separator of the type applicable for holding and separating the skin once the incision has been performed, characterised in that it is essentially formed by a rigid body formed by a rod (2), preferably in metal, which is formed by a cross segment (3) integrally joined at the ends to two separators (4) formed by branches (5) that extend from them at straight angles, which are parallel and on the same plane, and which are in turn provided at the ends with curved and sharp hook-shaped tips (6), which are both oriented in the same direction, this direction being perpendicular to the direction of the joints between the branches (5) and the segment (3).#2. A skin traction surgical separator according to claim 1, characterised in that the rod (2) has a circular or planar section.#3. A skin traction surgical separator according to claims 1 and 2, characterised in that the branches (5) forming the respective separators (4) and the segment (3) joining them are formed by independent parts that are integrally joined to one another or can be part of a single part or rod (2) that is bent at said points.#4. A skin traction surgical separator according to claims 1 to 3, characterised in that the length of the segment (3) joining the separators (4) and thus setting the distance between them can be variable, according to the needs in each case, whereas the length of each branch (5) of the respective separators (4), which will be the most suitable for the correct use of the instrument, is fixed (about 7 cm) and equal for both branches, and in that the diameter of the hook (6) at the end of each branch (5) of the separator (1) is also equal in both cases.

## Description

### Object of the invention

This invention, as its title suggests, relates to a skin traction surgical separator that provides several advantages and innovative features to its intended function, features that will be described in detail below and which entail important improvements to the state of the art in this field.

More specifically, the object of the invention consists in a surgical instrument that is especially applicable for holding and separating the skin once the incision has been performed, which advantageously has the special feature of being especially configured and designed, in a simple and effective manner, to form a rigid and fixed, i.e. non-articulated, double separator system, to which end it is formed by two atraumatic separators that allow simultaneously pulling from two points in the wound and lifting a segment thereof, the size of which will be determined by the separation existing between these points, providing notable advantages both in the reduction of the degree of trauma occurring in the skin, resulting in better wound healing, and in the surgeon's and/or team's own convenience, by reducing the number of instruments to be used and therefore resulting in greater freedom of movement.

### Background of the invention

It is known that the result of surgical activity is intimately related, among other things, to the satisfactory healing of the surgical wound performed, which in turn largely depends on having performed a correct handling of the tissues, especially at the dermo-epidermal anatomical level.

This is true not only in certain surgical subspecialties such as plastic surgery or dermatology, where the skin is in itself an organ object of the treatment, but also in the rest of surgical specialties where the need to perform incisions and surgical wounds is a common factor in order to access the inside of the body to handle specific organs or tissues.

In this context, adequate handling of the tissues involves making the least possible damage to the tissue and its different sources of vascularisation, i.e. the pathways of oxygen and nutrient supply to the skin. The interruption of this vascularisation or the performance of traumatic gestures that directly or indirectly damage the dermo-epidermal junction are responsible for more or less severe healing disorders that will at least cause aesthetical problems. Moreover, these problems can be associated with complications such as surgical would contamination and infection, increasing associated morbidity.

In the current state of the art the traditional handling of the dermoepidermal junction after skin incision is by the use of different types of forceps, which can be more or less traumatic (toothed or not), which at the least mean local pressure being repeatedly applied on the tissue.

Moreover, traction with this type of forceps entails the separation of a single or several points in the wound, but with the need to use several forceps, the help of one or more assistants performing the same operation thus being essential.

The use of "hook" type surgical separators or skin hooks partially solves the problem of tissue trauma, but not that of separation at a single point.

In addition to all that above is the need, in certain interventions that are becoming ever more frequent, of designing or defining dermoepidermal or myocutaneous flaps or grafts of different types, which all share the function of separating the dermoepidermal junction from the subcutaneous cellular tissue, creating a flap with variable thickness according to the type of information being performed, and during the creation of which it is essential to maintain adequate vascularisation (for the reasons mentioned above). This may be especially complex in the area where the wounds meet ("corners") where two flaps meet on the same section.

Thus, for example, in breast cancer surgery it is necessary to leave a tight skin thickness with the least amount of subcutaneous cellular tissue, where skin traction and separation from the underlying fat is an essential operation to perform in order to avoid, on the one hand, lesions from occurring in the deeper dermis that carries vascularisation, and on the other hand, to prevent leaving a thickness susceptible of containing tissue that may house a relapse of the malignant disease.

It is therefore necessary to create a surgical instrument for separation that allows, on the one hand, to adequately separate the skin in the terms described above, and on the other hand, to do this with the minimum aggression or trauma to the tissues handled, the main objective of the skin traction surgical separator object of the present invention being to cover both of these needs. Moreover, the applicant does not know of any other instrument with similar technical, structural and configurational features to those described herein.

### Description of the invention

Specifically, the skin traction surgical separator proposed by the invention consists in a rigid and non-articulated instrument formed by two separators which, each ending in a sharp and curved tip like a sharp hook, are oriented in the same direction and arranged parallel to one another, and are joined by a straight segment at a straight angle and on a same spatial plane.

With respect to the size of the separator, it is worth highlighting that the length of the branch or segment joining the hooks can be variable, and the choice will depend on the segment of the wound to be separated, whereas the length of each branch of the respective hooks forming the separator is fixed and equal for both hooks, and will be the most suitable for its intended use (about 7 cm). The diameter of the hook at the end of each branch of the separator is also equal in both books and about 1 cm in length.

Thus, in order to use the new separator, both hooks are anchored to the dermis once the surgical incision has been performed, allowing to traction from two points of the wound at the same time and lifting a segment thereof, the size of which will be determined by the separation existing between both hooks.

The traction applied by the surgeon with the non-dominant hand or by the assistant is performed perpendicularly to the plane of the skin, thus achieving a comfortable dissection and adequate separation of the most superficial plane with respect to the subdermal plane.

This separation allows the surgeon to operate more deeply, separating the flap with the desired thickness and designing the dermoepidermal flap to the necessary size. Therefore, the flap thickness obtained is more homogeneous throughout the surface than that usually obtained by traction from a single point of the wound, which often involves a more stepped and less controlled dissection with respect to its thickness.

Another advantage of the new skin traction surgical separator is that it can prevent the excessive devascularisation that may occur at the points at which the dissection is closest to the deep dermal plane when performed by traction from a single point with tent-like surgical fields.

As a result, the surgical separator proposed by the invention thus constitutes an important novelty within its field of application, since it allows the specific use of an instrument allowing simultaneous traction from two points at a surgical wound and thus lifting a segment thereof and not a single point, as with single skin hook systems.

Similarly, the separator of the invention advantageously allows traction of the dermal plane by anchoring with hook systems, without compromising the tissues as with the use of forceps. Moreover, it can be handled by the surgeon simultaneously to dissection, allowing traction and counter-traction handling operations.

The separator described, as mentioned above, allows controlling dissection of the thickness of a dermoepidermal or skin-fat flap, as well as simultaneously performing an optimal separation of the two sides at an angle of the surgical wound where two flaps converge, without the need to traction in both directions at once, and with a greater range than that achieved by traction with a single hook.

Finally, it is worth highlighting that the separator of the invention allows handling and extending dermoepidermal and skin-fat flaps or grafts with minimum trauma to the dermal planes and therefore with minimum aggression to their vascularisation.

The new skin traction surgical separator therefore represents an innovative structure with structural and constitutive features unknown for this end, reasons that, together with its practical usefulness, provide sufficient reason for it to obtain the privilege of exclusiveness requested.

### Description of the drawings

In order to complement the description that is being made and with the object of aiding towards a better understanding of the features of the invention, attached to the present specification and as an integral part thereof is a set of drawings in which the following has been represented with an illustrative and non-limiting nature:

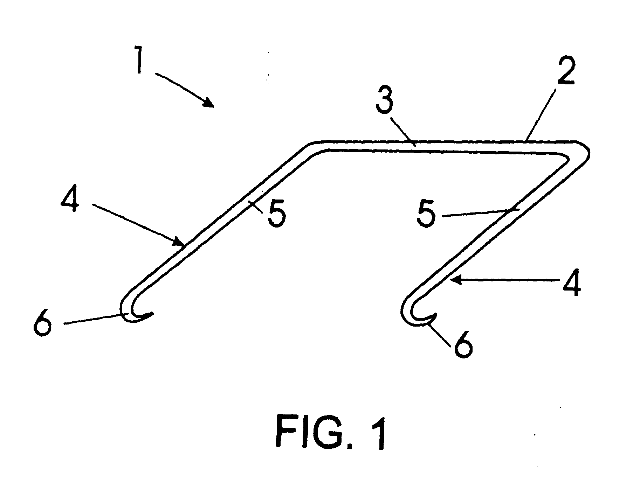

Figure 1.- Shows a perspective view of an embodiment of the new skin traction surgical separator object of the invention representing its main parts as well as its configuration and arrangement.

### Preferred embodiment of the invention

In view of Figure 1 described above and according to the numbering therein, the separator (1) can be observed to be formed essentially by a rigid body formed by a rod (2), preferably in metal, which can have a circular or planar section, which is formed by a cross segment (3) integrally joined at the ends to two separators (4) formed by branches (5) that extend from them at straight angles, which are parallel and on the same plane, and which are in turn provided at the ends with curved and sharp hook-shaped tips (6), which are both oriented in the same direction, this direction being perpendicular to the direction of the joints between the branches (5) and the segment (3).

It is worth mentioning that said branches (5) forming the respective separators (4) and the segment (3) joining them can be formed by independent parts that are integrally joined to one another, as described above, or can be part of a single part or rod (2) that is bent at said points, as in the example represented in Figure 1.

In turn, the length of the segment (3) joining the separators (4) and thus setting the distance between them can be variable, according to the needs in each case, whereas the length of each branch (5) of the respective separators (4), which will be the most suitable for the correct use of the instrument, is fixed, being about 7 cm and equal for both branches. The diameter of the hook (6) at the end of each branch (5) of the separator (1) is also equal in both cases.

Having sufficiently described the nature of the present invention and an embodiment thereof, it is not considered necessary to further extend its description for any person skilled in the art to have enough information to understand its scope and the advantages derived from it, as well as to be able to reproduce it. Also, it is worth noting that while maintaining its essential features it can lead to other embodiments that may differ in the details to the one indicated as an example, and which will be equally affected by the protection conferred by this patent, as long as its essential features are not altered, changed or modified.

## Claims

1. A skin traction surgical separator of the type applicable for holding and separating the skin once the incision has been performed, **characterised in that** it is essentially formed by a rigid body formed by a rod (2), preferably in metal, which is formed by a cross segment (3) integrally joined at the ends to two separators (4) formed by branches (5) that extend from them at straight angles, which are parallel and on the same plane, and which are in turn provided at the ends with curved and sharp hook-shaped tips (6), which are both oriented in the same direction, this direction being perpendicular to the direction of the joints between the branches (5) and the segment (3).

2. A skin traction surgical separator according to claim 1, **characterised in that** the rod (2) has a circular or planar section.

3. A skin traction surgical separator according to claims 1 and 2, **characterised in that** the branches (5) forming the respective separators (4) and the segment (3) joining them are formed by independent parts that are integrally joined to one another or can be part of a single part or rod (2) that is bent at said points.

4. A skin traction surgical separator according to claims 1 to 3, **characterised in that** the length of the segment (3) joining the separators (4) and thus setting the distance between them can be variable, according to the needs in each case, whereas the length of each branch (5) of the respective separators (4), which will be the most suitable for the correct use of the instrument, is fixed (about 7 cm) and equal for both branches, and **in that** the diameter of the hook (6) at the end of each branch (5) of the separator (1) is also equal in both cases.
